## Europäisches Patentamt
## European Patent Office
## Office européen des brevets

(19)

(11) Publication number: **0 052 511**
**B1**

(12)

# EUROPEAN PATENT SPECIFICATION

(45) Date of publication of the patent specification:
07.03.84

(51) Int. Cl.³: **C 07 C 87/58,** C 07 C 85/11, C 07 C 107/06

(21) Application number: 81305429.3

(22) Date of filing: 17.11.81

(54) Production of p-phenylenediamine.

(30) Priority: 17.11.80 US 207619
06.11.81 US 318191

(43) Date of publication of application:
26.05.82 Bulletin 82/21

(45) Publication of the grant of the patent:
07.03.84 Bulletin 84/10

(84) Designated Contracting States:
DE FR GB IT NL

(56) References cited:
DE - B - 2 216 117
GB - A - 891 234
US - A - 1 589 936

(73) Proprietor: **E.I. DU PONT DE NEMOURS AND COMPANY, Legal Department 1007 Market Street, Wilmington Delaware 19898 (US)**

(72) Inventor: **Rolston, Charles Hopkins, 24 Budd Boulevard, Woodbury New Jersey 08096 (US)**

(74) Representative: **Barnard, Eric Edward et al, BROOKES & MARTIN High Holborn House 52/54 High Holborn, London WC1V 6SE (GB)**

ACTORUM AG

## Production of p-Phenylenediamine

The present invention relates to the production of p-phenylenediamine (PPD) by the catalytic hydrogenation of p-aminoazobenzene (PAAB).

p-Phenylenediamine is an intermediate in the preparation of polymers, for example, all-aromatic polyamides. One method of preparing this diamine involves the diazotization-coupling of aniline to form 1,3-diphenyltriazene, and the rearrangement of the latter to form p-aminoazobenzene, which then is reduced to form p-phenylenediamine and aniline.

U.S. Patent 4,020,052, issued April 26, 1977, describes an improved method of preparing 1,3-diphenyltriazene wherein ammonia is catalytically oxidized so as to form a gas mixture containing dilute nitrogen dioxide preferably admixed with nitric oxide, and this gas mixture is contacted with excess aniline. U.S. Patent 4,020,051 describes a method of preventing the accumulation of potentially hazardous benzenediazonium nitrate in lines for conducting residual gases from aniline diazotization-coupling reactions.

In the method of U.S. Patent 4,020,052 the reaction product obtained from aniline is comprised of 1,3-diphenyltriazene dissolved in aniline. A method of rearranging the triazene in this solution to p-aminoazobenzene in a high para/ortho isomer ratio with minimum formation of polynuclear by-products is described in U.S. Patent 4,279,815. In this rearrangement method, the aniline solution containing up to about 30 percent by weight of the triazene is contacted with about from 0.4 to 2.0 percent by weight of a strong acid, preferably nitric acid, at a temperature in the range of about from 50° to 120°C at which the solution is substantially homogeneous. Although the p-aminoazobenzene can be recovered from the rearrangement reaction product, when the rearrangement constitutes a step in an overall p-phenylenediamine synthesis it naturally is preferred that the remainder of the synthesis, i.e., the reduction of p-aminoazobenzene to the diamine, be carried out without the need for isolating the amino azo compound.

Chemical reduction methods, as well as catalytic hydrogenation, have heretofore been described for reducing p-aminoazobenzene to the diamine. Reduction by means of iron and a mineral acid is described in U.S. Patent 2,708,680, according to which aniline is converted to 1,3-diphenyltriazene by reaction with aqueous sodium nitrite and hydrochloric acid, the triazene in the reaction mixture rearranged to the aminoazobenzene, and the latter reduced by adding iron and water to the rearrangement reaction mixture. Catalytic methods which have been described apply to the hydrogenation of aminoazobenzene in solution, but not in rearrangement reaction mixtures from which the aminoazobenzene has not been separated. For example, Andrews et al, Journal of the American Chemcial Society, Vol. 56, pages 1411–1412 (1934), describe reducing PAAB to PPD by treating a solution of PAAB in alcohol with hydrogen in the presence of platinum oxide. The hydrogenation described in Japanese Application Publication J 54003-018 wherein cobalt, nickel, or copper chromite is used as a catalyst, is performed on an aniline solution of PAAB. According to the process described in U.S. Patent 4,191,708, PAAB is first purified and recovered before undergoing catalytic hydrogenation. The use of nickel and palladium catalysts for the hydrogenation of p-azo N,N-disubstituted anilines is described in British Patent 891,234. Nickel is described also as catalyzing the hydrogenation of azo to hydrazo compounds (U.S. Patent 1,589,936).

A typical reaction product obtained from the triazene rearrangement described in U.S. Patent 4,279,815 is a two-phase liquid at temperatures in the range of about 25–40°C, i.e., the product has an organic phase consisting largely of aniline having dissolved therein PAAB and water; and a separate aqueous phase, the weight ratio of organic to aqueous phase typically being about 25/1. The dissolved water may constitute about 3–4 percent by weight of the organic phase. Both phases contain in solution the anion of the strong acid, usually nitric acid, used in the rearrangement. The product's content in the anion derived from the strong acid, e.g., the nitrate ion content, generally is up to about 1–2% by weight.

Naturally, any process employed to reduce the PAAB in such a product to PPD has to be specifically designed with the consideration in mind of interactions that may occur when the materials used for the reduction are combined with the product containing the PAAB. In a catalytic hydrogenation, for example, interactions could occur that would deleteriously affect a given catalyst, which would not be so affected if the PAAB were being hydrogenated in an essentially neat solvent.

The present invention provides a method of making p-phenylenediamine which comprises contacting a solution of p-aminoazobenzene (PAAB) in aniline with hydrogen in the presence of a nickel catalyst at a temperature of not more than 130°C (and generally a temperature in the range of about from 100° to 130°C), said solution containing dissolved water in an amount up to about 6, and preferably up to about 4, per cent by weight and anions derived from a strong acid in an amount less than about 500 parts per million by weight, and having substantially no free water admixed therewith.

The PAAB solution which is contacted with hydrogen according to the present process is obtained by

a) separating into an organic phase and an aqueous phase a product obtained by contacting a solution of 1,3-diphenyltriazene in aniline with about from 0.4 to 2.0 percent by weight of a strong acid, preferably nitric acid, said product being, at temperatures in the range of about from 25° to 40°C, a two-phase liquid having (1) an organic phase comprising aniline having dissolved therein p-aminoazobenzene and water, and (2) an aqueous phase, said organic phase and said aqueous phase both containing dissolved strong acid, preferably nitric acid, said product, optionally, having been previously neutralized, and said separation being conducted at a temperature in the range of about from 25° to 40°C and leaving an organic phase having a lower concentration of the anion derived from the strong acid, e.g., the nitrate ion, than said two-phase liquid, e.g., less than about 5000 parts per million, by weight;

b) contacting the organic phase separated in Step (a), at a temperature in the range of about from 25° to 40°C, with an aqueous solution of a base selected from the group consisting of ammonium hydroxide, ammonium carbonate, and alkylamines, whereby the product obtained has an aqueous phase containing a dissolved salt formed by the reaction of the base with strong acid, and an organic phase having a lower concentration of said anion than the organic phase separated in Step (a), i.e., less than about 500 parts per million, by weight; and

c) separating the organic and aqueous phases of the Step (b) product.

The organic phase of the Step (b) product separated in Step (c) contains substantially no free water but contains a few percent, e.g., up to about 6%, and usually up to about 4%, dissolved water.

The p-aminoazobenzene in the organic phase of the Step (b) product separated in Step (c) is ready for conversion to p-phenylenediamine by the catalytic hydrogenation process of the invention. Therefore, following Step (c), the organic phase is contacted with hydrogen in the presence of a nickel catalyst, preferably nickel supported on an aluminosilicate substrate, at a temperature in the range of about from 100°C to 130°C, preferably about from 115°C to 125°C. The activity (i.e., percent PAAB converted to products) and productivity (i.e., weight of PPD obtained per unit of catalyst) of the catalyst are higher when the organic phase of the Step (b) product is used in the hydrogenation, as contrasted to the use of the two-phase rearrangement liquid or the organic phase separated in Step (a).

The present invention is based on the finding that a nickel catalyst used in the hydrogenation of PAAB in a liquid product obtained from the rearrangement of 1,3-diphenyltriazene, which product contains aniline, water, and a strong acid, exhibits an increase in activity and productivity when the strong acid anion concentration in the liquid is reduced, free water is removed, and a small amount of water is present in solution in aniline.

The PAAB solution used in the process of this invention is the product of the rearrangement reaction described in U.S. Patent 4,279,815. As mentioned previously, at temperatures in the range of about from 25° to 40°C, this material is a two-phase liquid having (1) an organic phase comprising aniline having p-aminoazobenzene and water dissolved therein and (2) an aqueous phase, both phases in this product containing dissolved therein the strong acid, preferably nitric acid, used in the rearrangement method, the concentration of the anion derived from this acid typically being up to about 1–2% by weight. This two-phase liquid is separated into an organic phase and an aqueous phase, preferably by decantation. The liquid product obtained from the rearrangement, optionally after having been neutralized, can be allowed to sit in a settling tank to permit the organic and aqueous phases to separate, and the aqueous phase drawn off from the bottom of the tank. The purpose of this separation is to obtain an organic product having substantially no free water and a reduced content in the anion derived from the strong acid. For this reason, the separation is conducted at as low a temperature as is convenient, e.g., in the range of about from 25° to 40°C. Temperatures as high as 60°C should be avoided. If higher temperatures are used, acid-bearing water will dissolve in the aniline only to separate out again when the organic phase, after the separation, returns to ambient temperature.

Neutralization of the two-phase liquid rearrangement product prior to the just-described separation, e.g., by the addition of an aqueous alkali metal hydroxide or ammonium hydroxide solution, may be employed to provide a larger density difference for a faster separation of the phases.

The separated organic phase, which has substantially no free water and a typical strong acid anion concentration therein (whether or not the two-phase liquid has previously been neutralized) of less than about 5000, usually about 2000–4000, parts per million, by weight, is contacted with an aqueous solution of a base selected from the group consisting of ammonium hydroxide, ammonium carbonate, and alkylamines, which reacts with the strong acid to form a salt that is extracted into a separate aqueous phase, leaving the organic phase with a lower strong acid anion concentration, i.e., less than about 500 parts per million, by weight, and dissolved water in the range of about from 1 to 6 percent by weight, depending on the extraction temperature. In most cases, a single extraction will reduce the strong acid anion concentration to about 200–300 parts per million, and two extractions to about 50 parts per million or less. Low concentrations of base extractant, e.g., less than about 10 percent by weight, are effective. For example, removal of more than about 90 percent of the strong acid can be readily achieved with base concentrations of about from 1 to 5 percent. The small amount of base not only reacts with the acid but seems to contribute to a clean, rapid separation of the organic and aqueous phases. Ammonium hydroxide is a preferred base because of its compatibility with the remainder of the process including waste disposal. Maintaining the liquid below about 40°C during the extraction creates a sufficient phase density difference to allow rapid separation.

The small amount of dissolved water left in the organic phase after the extraction step, e.g., about from 1 to 6 percent, and usually about from 3 to 4 percent, by weight, has a beneficial effect on catalyst activity in the hydrogenation despite the fact that free water has a deleterious effect.

The organic phase obtained in the above-described extraction step is separated from the aqueous phase and is ready to be used in the present process for converting the PAAB therein to PPD by catalytic hydrogenation. The organic phase is contacted with hydrogen in the presence of a nickel catalyst, preferably nickel supported on an aluminosilicate substrate. The strong acid anion concentration of the liquid that contacts the catalyst preferably is less than about 200, and most preferably less than about 50, parts per million, and this can be provided by the organic phase per se separated after the extraction, or an organic phase of higher strong acid concentration can be used diluted with aniline or with a recycle stream from a previous hydrogenation having a sufficiently low strong acid anion concentration as to result in a low enough concentration in the combined liquids.

The hydrogenation can be carried out over a wide range of temperatures and pressures. However, inasmuch as lower pressures and temperatures cause the reaction rate to be low, it generally will be desirable to use a temperature of at least about 100°C and a pressure of at least about 2070 kPa. A preferred temperature range is about from 115° to 125°C. Temperatures above about 130°C result in yield losses due to ring hydrogenation, and therefore are not useful. Pressures as high as about 5000 kPa or more can be used but appear to produce little or no improvement in the hydrogenation rate.

During the hydrogenation, the reactor feed to which the catalyst is exposed contains substantially no free water, has a low concentration of the anion derived from a strong acid, i.e., less than about 500 parts per million, by weight, and contains a few percent, e.g., up to about 6%, dissolved water, by weight.

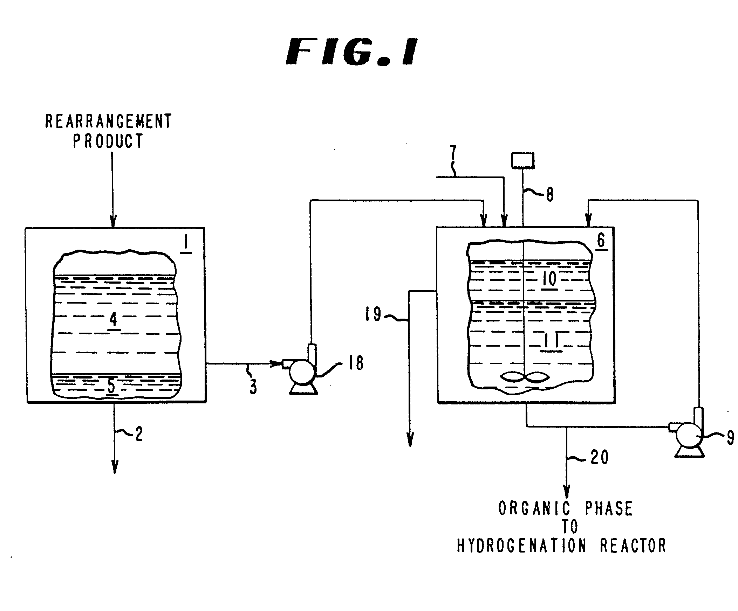

A method of treating a 1,3-diphenyltriazene rearrangement product to adapt it for use in the catalytic hydrogenation of PAAB to PPD according to the present invention will now be illustrated by means of an example and the schematic diagram shown in Fig. 1.

Example 1

A 1,3-diphenyltriazene rearrangement product was prepared by the procedure described in Example 1 of U.S. Patent 4,279,815, the disclosure of which is incorporated herein by reference. This procedure was as follows:

I. Preparation of 1,3-diphenyltriazene (according to U.S. Patent 4,020,052)

a) Synthesis of nitrogen oxide-containing gas mixture

Nitric oxide and air were fed into a pipeline reactor at 648 kPa at rates of $0.57 \times 10^{-4}$ and $0.61 \times 10^{-4}$ m³/s, respectively, with a hold-up time sufficient to allow the reaction of 95–97% of the oxygen fed and to produce a gas mixture containing equal volumes of nitrogen dioxide and nitric oxide. Heated nitrogen was added to the product gas at a rate of $0.39 \times 10^{-3}$ m³/s to give a final stream containing 10.8 mole percent nitrogen oxides at a temperature of 74°C, and less than 0.14 mole percent residual oxygen. This gas mixture, except for water, simulated that which would be produced by burning 12.3% ammonia in air of 80% relative humidity at 30°C.

b) Reaction of nitrogen oxides with aniline

Aniline and water [the latter in the amount which would be present in the ammonia oxidation process gas described in Paragraph (a)] were added, at rates of 108 grams/minute and 4.3 milliliters/minute, respectively, through a water-cooled condenser and thence into a vapor-liquid separator designed for continuous drawoff. The reaction product solution (1,3-diphenyltriazene dissolved in aniline) and gases produced in a tubular reactor also passed into the separator, liquid being withdrawn from the separator continuously to maintain a constant level. Part of the withdrawn liquid was recovered, and part of it was recirculated through a cooler to maintain a temperature of 40–50°C, and then to the tubular reactor at a rate of about 2300 milliliters per minute.

The nitrogen oxide-containing gas stream produced as described in Paragraph (a) was passed through the reactor cocurrently with the recirculated aniline solution stream, and the product (liquid and gas) passed into the separator as described above.

Analysis of the liquid recovered from the separator indicated the following composition, by weight:

| | |
|---|---|
| 1,3-Diphenyltriazene | 19.3% |
| Aniline | 78.0% |
| p-Aminoazobenzene | 1.3% |
| o-Aminoazobenzene | 0.05% |
| Aminobiphenyls | 0.022% |
| Nitric acid | 0.12% |
| Water | 1.00% |

II. Rearrangement of 1,3-diphenyltriazene

The above-described essentially homogeneous triazene-containing reaction solution obtained by the diazotization-coupling of aniline as described in Part I (above) was allowed to flow at a rate of 112 milliliters per minute through a 1.8 meter-long, 1.09-cm inner diameter preheater tube, the first 0.34 meter of which was packed with fine wire mesh so as to constitute a mixing zone. The temperature in the preheater tube was 40°C. At the same time, a 20 percent by weight solution of nitric acid was introduced into the mixing zone of the preheater tube at a rate of 3.6 milliliters/minute. The total amount of nitric acid contacted with the aniline solution was 0.9 percent of the weight of the solution. The mixture leaving the preheater flowed through 12 meters of 2.3-cm outer diameter stainless steel tubing (1.09-cm inner diameter) at a rate of 116 milliliters/minute, providing a hold-up time in the reactor tubing of 10 minutes (1145 cc reactor volume). Hot water was circulated through a jacket around the reactor tubing to maintain a reaction temperature of 85°C, as indicated at the reactor exit. The reactor effluent (rearrangement product) contained, by weight, 75.5% aniline, 16.1% PAAB, and 0.8% nitrate ion.

III. Nitrate removal

a) Removal of free water

A 190-kg batch of the above rearrangement product was charged to decanter vessel 1 (Fig. 1), a 208-liter stainless steel tank equipped with bottom and side draw-off lines 2 and 3, respectively, and allowed to remain there at about 25°C for 4 hours, whereby the batch separated into an organic phase 4 and an aqueous phase 5. The aqueous phase (7 kg) was drained from the tank through line 2 until organic phase was observed in a sample thereof. The withdrawn aqueous phase contained, by weight, 3.64% aniline, 16.9% nitrate ion, and less than 0.1% PAAB. The organic phase in vessel 1 (183 kg) contained, by weight, 75.5% aniline, 3620 parts per million of nitrate ion, 16.1% PAAB, about 4% dissolved water, and substantially no free water.

b) Neutralization-extraction

The organic phase 4 was withdrawn from vessel 1 through line 3 and pumped via pump 18 into a 208-liter stainless steel extractor vessel 6 for the removal of residual nitrate ion (which could be accomplished in decanter 1, if desired). Also added to vessel 6, through line 7, were 39 kg of water and 2 kg of a 30% aqueous ammonium hydroxide solution. This amounted to adding 41 kg of a 1.7% ammonium hydroxide solution. The liquid in the extractor was agitated with stirrer 8 and recirculation pump 9 for 1 hour at 25°C, and then was allowed to settle for 4 hours, whereupon an aqueous phase 10 separated from an organic phase 11. The upper aqueous layer (43 kg) was suctioned off the organic layer (183 kg) via line 19. The aqueous phase contained, by weight, 3.2% aniline, 1.5% nitrate ion, and less than 0.1% PAAB. The organic phase contained, by weight, 75.4% aniline, 16.1% PAAB, and about 4% dissolved water, and was left with only 220 parts per million of nitrate ion. Repetition of the extraction step reduced the nitrate ion content at least to about 50 parts per million.

The following example illustrates the PAAB hydrogenation method of the invention.

Example 2

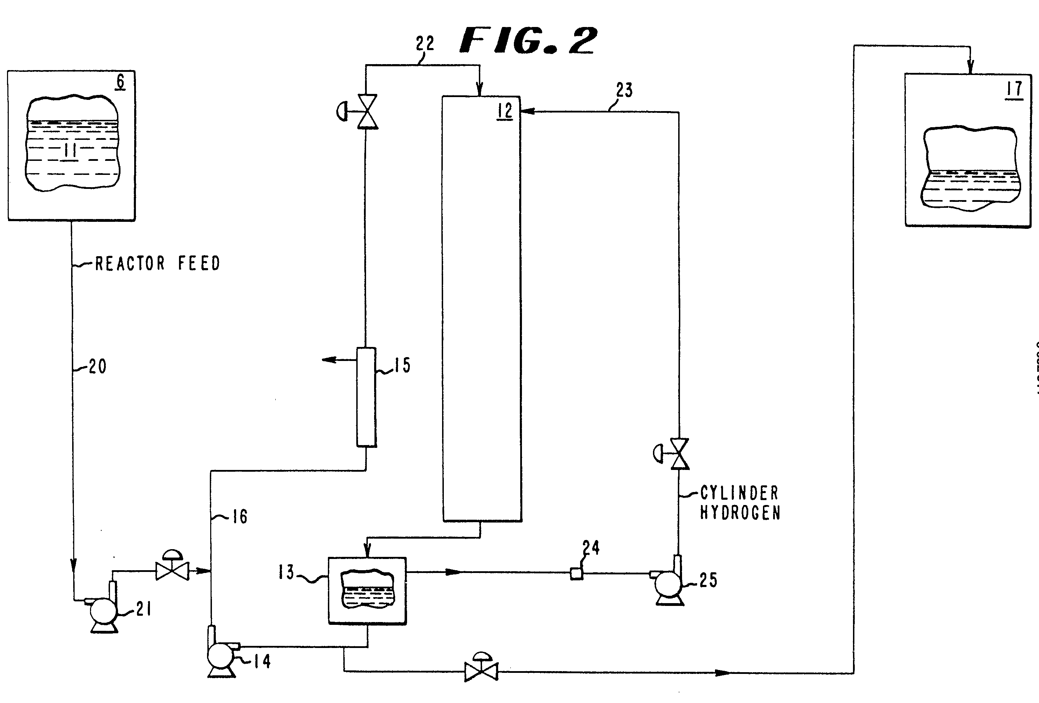

The PAAB in the organic phase containing 220 ppm of nitrate ion, obtained as described in Example 1, was hydrogenated by contacting the organic phase with hydrogen and a nickel catalyst. A vertical reactor 12 (Fig. 2), 5 cm in diameter and 189 cm long, was packed with 1.4 kg of a 3.18-mm diameter, extruded 50% nickel-on-aluminosilicate catalyst. The PAAB-containing organic liquid 11 and hydrogen were fed cocurrently at the top of the reactor through lines 22 and 23, respectively, and allowed to trickle down through the catalyst bed, exiting from the bottom of the reactor and entering a gas-liquid separator 13. Some of the liquid leaving the separator was recycled to the reactor to limit the temperature rise from the top to the bottom of the reactor to 5–10°C.

The reactor was pressurized to 3310 kPa with hydrogen, and liquid 11, which left feed tank 6 (the extractor vessel of Example 1, Part IIIb) through line 20, was pumped into reactor 12 to establish a liquid level of 50–70% of the total volume in separator 13. Recycle pump 14 was turned on, and the flow of liquid 11 was stopped to allow stabilization of system temperatures using heater 15 in recycle line 16. Once the reactor inlet was at 115°C, feed of liquid 11 was begun, and the product was continuously removed to maintain a constant separator level. The reactor exit temperature was 120°C. The hydrogen leaving the gas-liquid separator was recycled to the reactor via pressure regulator 24 and hydrogen compressor 25 with cylinder hydrogen being introduced into line 23 to make up for consumed hydrogen.

Liquid 11 was pumped into recycle line 16 via pump 21 at a rate of 7.7 kg per hour, and the combined fresh and recycle streams were heated to 118°C in heater 15 and fed into reactor 12 at a rate of 92.5 kg per hour. The combined streams contained 2.3% PAAB, 0.30% OAAB, 7.3% PPD, 82.6% aniline, 4% water, and 50 ppm nitrate ion by weight. Liquid product from separator 13 was recovered in product tank 17 at a rate of 7.7 kg per hour. It contained 8% PPD, 4% $H_2O$, 1% PAAB, and 50 ppm nitrate ion by weight. The PPD was separated from the product by distilling off the aniline and water.

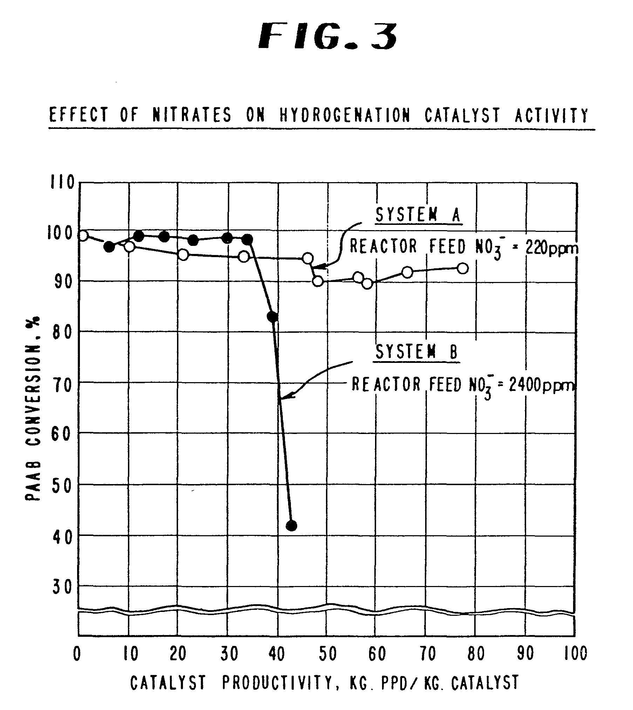

Fig. 3 shows the level and duration of catalyst activity in the above-described hydrogenation system (A), wherein the liquid contacted with the catalyst contained 50 ppm of nitrate ion (220 ppm in the reactor feed), in contrast to those achieved with the same system wherein the liquid contacted with the catalyst contained 600 ppm of nitrate ion (System B). In System B, liquid 11 contained 240 ppm of nitrate ion, not having been subjected to the extraction procedure according to the present process. The nitrate level of this liquid was reduced to 600 ppm by combining with the described recycle stream. In System B, 3.1 kg of catalyst was used.

As can be seen from Fig. 3, the conversion level of PAAB to products in System A remained high (i.e., in the 90–100% range) over a period during which 76 kg of PPD had been produced per kg of catalyst, and furthermore showed no signs of sudden drop with further productivity. In System B, on the other hand, the catalyst had failed (i.e., the conversion level had dropped drastically) by the time about 40 kg of PPD had been produced per kg of catalyst.

Example 3

Two hydrogenation catalyst life and activity experiments were carried out in a stirred autoclave with the same catalyst described in Example 2, except in slurry form, and with productivity measurements extended to 320 kg PPD per kg catalyst. Each experiment was a repetitive, cyclic test in which the same charge of catalyst was reused in succeeding batch slurry hydrogenation cycles with a feed charge, by weight, of (a) 60% fresh rearrangement product and (b) 40% heel from the previous cycle. The two experiments were made under identical conditions, differing only in the composition of the rearrangement product in the feed charge, i.e., one having been treated by the nitrate-reducing process of the invention and the other not.

Experiment 3A used as the feed a rearrangement product made as described in Example 1, Part II, and treated as described in Example 1, Part III, a and b. In this case, the organic phase 11, after removal of aqueous layer 10 therefrom, was dried, and diluted 1 to 1 with virgin aniline to give a reactor feed containing 9.7% PAAB, 1.4% OAAB, 0% PPD, 88% aniline, 0.2% water, and less than 25 ppm nitrate ion. Experiment 3B (control) used as the feed a rearrangement product made as described in Example 1, Part II which had been diluted 1 to 1 with virgin aniline and contained about 11% total aminoazobenzenes, 2% water, and 1000 ppm nitrate ion.

Each hydrogenation cycle was carried out as follows:

One hundred fifty milliliters of the triazene rearrangement product, treated as described above, and 0.50 gram of the catalyst described in Example 2, ground to a size such that all particles passed through a U.S. No. 325 sieve (0.044 mm opening), were charged to an electrically heated 30-milliliter stirred stainless steel autoclave equipped with a gas-recirculating turbine agitator, thermostatically controlled cooling coils, and charging and product-withdrawal lines to permit operation on a semi-continuous basis without the need for opening the autoclave. The system was purged with nitrogen, and then hydrogen.

The autoclave was heated to 120°C under 3550 kPa hydrogen with sufficient agitation to keep the catalyst suspended but to minimize hydrogen uptake. A hydrogen reservoir of known volume was adjusted to 3550 kPa, closed off from the rest of the gas feed system and agitation increased to 1250 rpm to signal the start of the reaction period.

As soon as full agitation was started, a steady pressure drop indicative of hydrogen uptake commenced and continued while the aminoazobenzenes were being converted to the phenylenediamines. The temperature was maintained at 120±0.5°C throughout the cycle by adjusting the flow of cooling water through the reactor coils.

When the hydrogen uptake had ceased, the reaction mixture was cooled to below 50°C and hydrogenate removed from the reactor, about 40% by weight having been retained in the reactor as a heel for the next hydrogenation cycle. This retention was necessitated by the location of a catalyst filter in the exit line above the agitator blades. The system was vented and fresh rearrangement product feed, equivalent in weight to the hydrogenate removed, was charged to the reactor, whereupon the hydrogenation sequence was repeated.

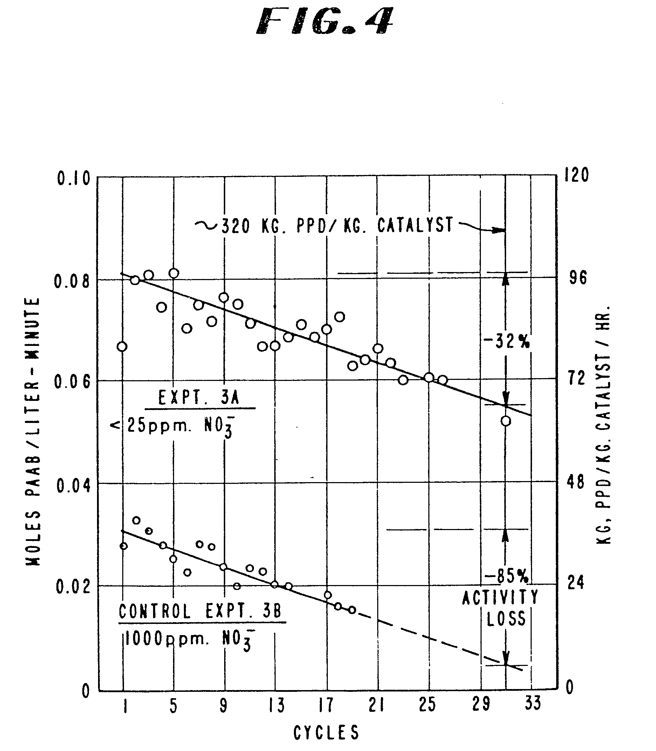

Repetitive hydrogenations with a given catalyst charge were continued until an adequate measure of performance was obtained or the catalyst failed. The results are shown in Fig. 4. A direct indication of catalyst activity was provided by the rate of hydrogen uptake, a measure of PAAB conversion rate expressed in Fig. 4 as moles of PAAB per liter-minute. The conversion (consumption) of the aminoazobenzenes was 100% in all cases.

Fig. 4 shows that the catalyst activity decreased 32% over 31 cycles in Experiment 3A, whereas in Control Experiment 3B the loss in catalyst activity in 31 cycles was 85%. (Note: Control Experiment 3B was concluded with Cycle 19 because adequate indications of rate trends and catalyst life had been established by then. Therefore, the loss in catalyst activity in 31 cycles for this experiment was based on extrapolation.) The catalyst productivitiy at the end of the 31 cycles was about 320 kg p-phenylenediamine per kg of catalyst for Experiment 3A, whereas it was only 196 kg for the Control experiment.

**Claims**

1. A method of producing p-phenylenediamine which method comprises contacting a solution of p-aminoazobenzene in aniline with hydrogen in the presence of a nickel catalyst at a temperature of not more than 130°C, said solution containing dissolved water in an amount up to 6 per cent by weight and anions derived from a strong acid in an amount less than 500 parts per million by weight, and having substantially no free water admixed therewith.

2. A method as claimed in Claim 1 wherein said anions are nitrate ions.

3. A method as claimed in Claim 1 or Claim 2 wherein said catalyst is nickel supported on an aluminosilicate substrate.

4. A method as claimed in any one of Claims 1 to 3 wherein said p-aminoazobenzene solution is one which has been obtained by a procedure comprising the following steps, namely:

a) separating into an organic phase and an aqueous phase a product obtained by contacting a solution of 1,3-diphenyltriazene in aniline with from 0.4 to 2 per cent by weight of a strong acid, said product being, at temperatures in the range of from 25° to 40°C, a two-phase liquid having (1) an organic phase comprising aniline having dissolved therein p-aminoazobenzene and water, and (2) an aqueous phase, said organic phase and said aqueous phase both containing dissolved strong acid, said separation being conducted at a temperature in the range of from 25° to 40°C and leaving an organic phase having a lower concentration of the anion derived from said strong acid than said two-phase liquid;

b) contacting the organic phase separated in Step (a), at a temperature in the range of from 25° to 40°C, with an aqueous solution of a base selected from ammonium hydroxide, ammonium carbonate, and alkylamines, whereby a product is obtained having an aqueous phase containing a dissolved salt formed by the reaction of said base with strong acid, and an organic phase having a lower concentration of said anion than the organic phase separated in Step (a); and

c) separating the organic and aqueous phases of the Step (b) product, said organic phase separated in Step (c) constituting the p-aminoazobenzene solution contacted with hydrogen.

5. A method as claimed in Claim 4 wherein the two-phase liquid separated in Step (a) contains nitric acid in the aqueous phase and in the organic phase thereof.

6. A method as claimed in Claim 5 or Claim 6 wherein the organic phase separated in Step (a) is contacted with an aqueous solution of ammonium hydroxide.

7. A method as claimed in Claim 6 wherein the concentration of said ammonium hydroxide solution is in the range of from 1 to 5% by weight.

8. A method as claimed in any one of Claims 4 to 7 wherein said two-phase liquid separated in Step (a) is neutralized prior to said separation.

**Revendications**

1. Un procédé de production de p-phénylène-diamine, qui comprend la mise en contact d'une solution de p-aminoazobenzène dans de l'aniline avec de l'hydrogène en présence d'un catalyseur au nickel à une température de pas plus de 130°C, cette solution contenant de l'eau dissoute à raison de jusqu'à 6 pour cent en poids et des anions dérivés d'un acide fort à raison de moins de 500 parties par million en poids, et n'ayant sensiblement pas d'eau libre mélangée avec elle.

2. Un procédé selon la revendication 1, dans lequel les anions sont des ions nitrate.

3. Un procédé selon la revendication 1 ou la revendication 2, dans lequel le catalyseur est du nickel déposé sur un support d'aluminosilicate.

4. Un procédé selon l'une quelconque des revendications 1 à 3, dans lequel la solution de p-aminobenzène est une solution qui a été obtenue par un mode opératoire comprenant les étapes suivantes:

a) on sépare en une phase organique et une phase aqueuse un produit obtenu par mise en contact d'une solution de 1,3-diphényltriazène dans l'aniline avec 0,4 à 2 pour cent en poids d'un acide fort, ce produit étant, à des températures comprises entre 25° et 40°, un liquide à deux phases ayant (1) une phase organique comprenant de l'aniline dans laquelle sont dissous du p-aminoazobenzène et de l'eau, et (2) une phase aqueuse, la phase organique et la phase aqueuse contenant toutes deux un acide fort dissous, la séparation étant effectuée à une température comprise entre 25° et 40°C et laissant une phase organique ayant une teneur moindre en l'anion dérivé de l'acide fort que le liquide à deux phases;

b) on met en contact la phase organique séparée dans l'étape (a), à une température comprise entre 25° et 40°C, avec une solution aqueuse d'une base choisie parmi l'hydroxyde d'ammonium, le carbonate d'ammonium et des alcoyl-amines, de façon que l'on obtienne un produit ayant une phase aqueuse contenant un sel dissous formé par la réaction de la base avec l'acide fort, et une phase organique ayant une moindre teneur en l'anion que la phase organique séparée dans l'étape (a); et

c) on sépare les phases organique et aqueuse du produit de l'étape (b), la phase organique séparée dans l'étape (c) constituant la solution de p-aminoazobenzène mise en contact avec l'hydrogène.

5. Un procédé selon la revendication 4, dans lequel le liquide à deux phases séparé dans l'étape (a) contient de l'acide nitrique dans sa phase aqueuse et dans sa phase organique.

6. Un procédé selon la revendication 5 ou la revendication 6, dans lequel la phase organique séparée dans l'étape (a) est mise en contact avec une solution aqueuse d'hydroxyde d'ammonium.

7. Un procédé selon la revendication 6, dans lequel la concentration de la solution d'hydroxyde d'ammonium est comprise entre 1 et 5% en poids.

8. Un procédé selon l'une quelconque des revendications 4 à 7, dans lequel le liquide à deux phases séparé dans l'étape (a) est neutralisé avant la séparation.

**Patentansprüche**

1. Verfahren zur Herstellung von p-Phenylendiamin durch Kontakt einer Lösung von p-Aminoazobenzol in Anilin mit Wasserstoff, in Anwesenheit eines Nickelkatalysators, bei einer Temperatur von nicht mehr als 130°C, wobei die Lösung gelöstes Wasser in einer Menge bis zu 6 Gew.-% und Anionen, abgeleitet von einer starken Säure in einer Menge von weniger als 500 Teilen pro Million bezogen auf das Gewicht, enthält, und im wesentlichen kein freies Wasser damit vermischt enthält.

2. Verfahren nach Anspruch 1, bei dem die Anionen Nitrationen sind.

3. Verfahren nach Anspruch 1 oder Anspruch 2, bei dem der Kastalysator Nickel, getragen auf einem Aluminosilicat-Substrat, ist.

4. Verfahren nach einem der Ansprüche 1 bis 3, bei dem die p-Aminoazobenzol-Lösung eine solche ist, die erhalten wurde durch ein Verfahren, das folgende Stufen umfasst, nämlich:

a) Trennen eines Produkts, das erhalten wurde durch Kontakt einer Lösung von 1,3-Diphenyltriazen in Anilin mit 0,4 bis 2 Gew.-% einer starken Säure, wobei das Produkt bei einer Temperatur im Bereich von 25° bis 40°C eine Zwei-Phasen-Flüssigkeit ist mit (1) einer organischen Phase, die Anilin aufweist, das p-Aminoazobenzol und Wasser gelöst enthält, und (2) einer wässrigen Phase, wobei die organische Phase und die wässrige Phase beide gelöste starke Säure enthalten, in eine organische Phase und eine wässrige Phase, wobei die Trennung bei einer Temperatur im Bereich von 25° bis 40°C durchgeführt wird, und eine organische Phase hinterlässt, die eine geringere Konzentration des Anions aufweist, das sich von der starken Säure ableitet, als die Zwei-Phasen-Flüssigkeit;

b) Kontakt der in der Stufe (a) abgetrennten organischen Phase, bei einer Temperatur im Bereich von 25° bis 40°C, mit einer wässrigen Lösung einer Base, ausgewählt aus Ammoniumhydroxid, Ammoniumcarbonat und Alkylaminen, wodurch ein Produkt mit einer wässrigen Phase, die ein gelöstes Salz, gebildet durch Reaktion der Base mit starker Säure, und eine organische Phase mit einer niedrigeren Konzentration des Anions als die in der Stufe (a) abgetrennte organische Phase enthält, erhalten wird; und

c) Trennen der organischen und wässrigen Phasen des Produkts der Stufe (b), wobei die in der Stufe (c) abgetrennte organische Phase, die mit Wasserstoff kontaktierte p-Aminoazobenzol-Lösung darstellt.

5. Verfahren nach Anspruch 4, bei dem die Zwei-Phasen-Flüssigkeit, die in der Stufe (a) abgetrennt wurde, Salpetersäure in der wässrigen Phase und der organischen Phase enthält.

6. Verfahren nach Anspruch 5 oder Anspruch 6, bei dem die in der Stufe (a) abgetrennte organische Phase mit einer wässrigen Lösung von Ammoniumhydroxid in Kontakt gebracht wird.

7. Verfahren nach Anspruch 6, bei dem die Konzentration der Ammoniumhydroxid-Lösung im Bereich von 1 bis 5 Gew.-% liegt.

8. Verfahren nach einem der Ansprüche 4 bis 7, bei dem die in der Stufe (a) abgetrennte Zwei-Phasen-Flüssigkeit vor der Abtrennung neutralisiert wird.

# FIG.1

REARRANGEMENT
PRODUCT

ORGANIC PHASE
TO
HYDROGENATION REACTOR

**FIG. 2**

# FIG. 3

EFFECT OF NITRATES ON HYDROGENATION CATALYST ACTIVITY

SYSTEM A
REACTOR FEED $NO_3^- = 220$ ppm

SYSTEM B
REACTOR FEED $NO_3^- = 2400$ ppm

PAAB CONVERSION, %

CATALYST PRODUCTIVITY, KG. PPD / KG. CATALYST

# FIG. 4